# EUROPEAN PATENT SPECIFICATION

(11) **EP 1 458 320 B1**
(45) Date of publication and mention of the grant of the patent: **21.06.2006**
(21) Application number: 02795242.3
(22) Date of filing: 19.12.2002
(51) Int. Cl.: A61F 13/20

(54) **TAMPON IN PARTICULAR FOR FEMININE HYGIENE**
TAMPON FÜR FRAUENHYGIENE
TAMPON DESTINE NOTAMMENT A L'HYGIENE FEMININE

(30) Priority: 24.12.2001 DE 10163114
(43) Date of publication of application: 22.09.2004
(73) Proprietor: JOHNSON & JOHNSON GmbH, 40474 Düsseldorf (DE)
(72) Inventor: KRÄMER, Robert, 51065 Köln (DE)
(74) Representative: Metten, Karl-Heinz
(86) International application number: PCT/EP2002/014592
(87) International publication number: WO 2003/055429

(56) References cited:
- WO-A-00/13638
- US-A- 2 123 750
- US-A- 4 217 900
- US-A- 4 714 466
- US-A- 5 911 712
- US-B1- 6 258 075

## Description

### Field of the Invention

The invention relates to a tampon, in particular for feminine hygiene, with an introduction end, with a recovery end, from which a withdrawal string extends, with a first region which extends from the introduction end in the direction of the recovery end and comprises a first material, and with a second region which is located near the recovery end of the tampon and comprises a second material, the first material having a higher absorbency and a higher hydrophilicity than the second material.

### Background of the Invention

In-vivo functionality tests have shown that most of the conventional tampons have a leakage before the theoretical maximum absorption capacity is exhausted. Many efforts have been made in the past in order to reduce said leakage.

Thus, for example, US 6,258,075 B 1 discloses generic tampons, in each case for use within the vaginal opening of a user, in which, in each case, a first region of the tampon extends from an introduction end as far as a recovery end of the tampon and a second region is located at the recovery end of the tampon, with a diameter perpendicular to the longitudinal extent of the tampon which is smaller than that of the first region, in particular at the recovery end. The second region is in this case intended essentially to prevent body fluid from being capable of penetrating as far as the withdrawal string, thus leading to a reduction of leakage. For this purpose, at least some fluid penetrating as far as the recovery end is to be forced, via the second region, to be directed back into the first region and absorbed there. Such a deflection of a fluid flow can be achieved, using a hydrophilicity gradient according to which the second region is less hydrophilic than the first region. One disadvantage is that a tampon according to US 6 258 075 B1 has only a reduction of the leakage via the withdrawal string, but without being efficient with regard to preventing a bypass leakage.

### Brief Description of the Invention

An object of the present invention is to provide a tampon with a structure that substantially reduces leakage from the tampon during use.

In accordance with one embodiment of the invention, a tampon, in particular for feminine hygiene, with an introduction end, with a recovery end, from which a withdrawal string extends is disclosed. The tampon has a first region which extends from the introduction end in the direction of the recovery end and comprises a first material, and with a second region which is located near the recovery end of the tampon and comprises a second material. The first material has a higher absorbency and a higher hydrophilicity than the second material. The diameter of the second region (essentially perpendicularly to the longitudinal axis of the tampon) is at least as large as the mean diameter of the first region of the tampon, and the second region expands at least essentially perpendicularly to the longitudinal axis of the tampon.

### Brief Description of the Drawing

The present invention will be more fully understood and further advantages will become apparent when reference is made to the following detailed description of the invention and the drawings, in which:

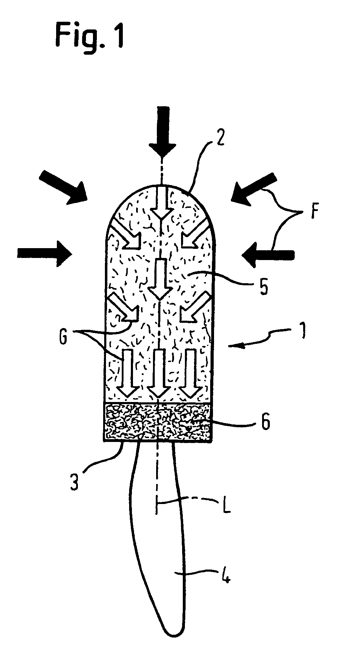

FIG. 1 illustrates a sectional view through a tampon according to the invention.

### Detailed Description of the Preferred Embodiments

The invention is thus based on the surprising finding that, in addition to the use of a hydrophilicity gradient over the length of a tampon for the purpose of increasing the quantity of fluid absorbed in a tampon and increasing the dwell time of fluid in the tampon, in that hydrophobic properties exist in the region of the recovery end of the tampon, the second region, when the tampon is placed in a vaginal opening, this second region also expands (swells up) in the event of the absorption of fluid, so that the vaginal opening can be closed off relative to the outside to an increased extent for the purpose of the reduction of bypass leakage. Assuming a tampon that has essentially the same diameter over its complete longitudinal extent before use and is introduced completely into a vaginal opening and fluid impinges onto the second region, part of the fluid is deflected in the direction of the first region. Another part of the fluid ensures an expansion of the second region that leads to a plug-like closure of the vaginal opening. Thus, according to the invention, the second region ensures leakage reduction in two respects.

Absorbent materials useful in the formation of the tampon include fiber, foam, superabsorbent, hydrogels, and the like. Preferred absorbent material for the present invention includes foam and fiber. Absorbent foams may include hydrophilic foams, foams that are readily wetted by aqueous fluids as well as foams in which the cell walls that form the foam themselves absorb fluid.

Preferably, the fibers employed in the formation of the tampon include regenerated cellulosic fiber, natural fibers and synthetic fibers. Preferably, the materials employed in the formation of a tampon according to the present invention include fiber, foam, hydrogels, wood pulp, and the like. A useful, non-limiting list of useful tampon fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the tampon. Preferably, tampon fibers are rayon or cotton, and more preferably, the fibers are rayon. The fibers may have any useful cross-section.

Fiber cross-sections include multi-limbed and non-limbed. Multi-limbed, regenerated cellulosic fibers have been commercially available for a number of years. These fibers are known to possess increased specific absorbency over non-limbed fibers. A commercial example of these fibers is the Danufil™ VY multilimbed viscose rayon fibers available from Acordis UK Ltd., Spondon, England. These fibers are described in detail in Wilkes et al., US Pat. No. 5,458,835 (EP 0 301 874), the disclosure of which is hereby incorporated by reference. Preferably, the fibers include hydrophilic fibers, and more preferably, the fibers include absorbent fibers, i.e., the individual fibers, themselves, absorb fluid. A useful, non-limiting list of useful tampon fibers includes natural fibers such as cotton, wood pulp, jute, hemp, and the like, and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the tampon. For example, hydrophobic fibers may be used in outer surfaces of the tampon to reduce surface wetness and hydrophilic fibers may be used to increase the rate of fluid transport into and throughout the body. Preferably, the tampon fibers are rayon or cotton, and more preferably, the fibers are rayon. The fibers may have any useful cross-section.

According to the figure, a preferred tampon 1 according to the invention comprises an introduction end 2 and a recovery end 3, from which a withdrawal string 4 extends. A first region 5 extends from the introduction end 2 in the direction of the recovery end 3, whilst a second region 6 is located at the recovery end 3 of the tampon 1. The tampon 1 has a longitudinal axis L, about which it is designed essentially cylindrically.

The first region 5 comprises a hydrophilic fibre mixture, composed, for example, of pressed cellulose fibres that have high absorbency and expand during the absorption of a fluid. In particular, fibres known under the name Galaxy™ or rather viscose of VY Type, available via Acordis Industrial Fibers B.V., Netherlands, are suitable for this purpose.

By contrast, the second region 6 comprises hydrophobic fibres, such as hydrophilic fibres treated with water repellant finishes; relatively hydrophobic fibers such as polyesters and polyamides; and substantially hydrophobic fibers such as polyolefins (e.g., polypropylene and polyethylene), or multicomponent fibers wherein one component such as polyester is enveloped by or is alongside with another component such as polyethylene, polypropylene or a lower-melting polyester. An example of treated hydrophilic fibres is cellulose fibres, obtainable, in particular, under the tradename Danufil™, which are coated with a hydrophobic material, in particular with the coating BK 2047FL from the company Henkel, and hydrophilic fibres such as cellulose fibres, like Galaxy.

In a preferred embodiment, the second region 6 is produced preferably as a pressed fibre nonwoven from approximately 70% by weight of hydrophobic fibres and approximately 30% by weight of hydrophilic fibres swelling up during the absorption of a fluid. Based upon this description, it will be recognized that the second region may be at least partially fluid-permeable.

In order to be used for feminine hygiene, the tampon 1 is to be introduced into a vaginal opening, not illustrated, in such a way that both its regions 5, 6 are arranged in the vaginal opening. When an absorption of fluid by the tampon 1 occurs as a result of a fluid flow F in the vaginal opening, the fluid is absorbed at least partially within the tampon 1, to be precise in the first region 5. In this case, a fluid gradient G also occurs within the first region 5, until the interface between the first region 5 and the second region 6 is reached. By virtue of the hydrophobic properties of the second region 6, part of the fluid reaching the interface is then deflected into the first region 5, so that the absorption capacity of the first region 5 can be fully utilized. This, on the one hand, increases the quantity of fluid absorbed in the tampon 1 and also the dwell time of fluid within the tampon 1. Moreover, during the absorption of fluid, an expansion of the second region 6 ensures that the diameter of the second region 6 perpendicularly to the longitudinal axis L of the tampon 1 is larger than that of the first region 5 and therefore an engagement of the tampon 1 onto the walls of the vaginal opening takes place with the result that the vaginal opening is essentially sealed off, in order to prevent, or at least markedly reduce, a bypass leakage. The second region 6 thus forma a barrier in two respects, to be precise, on the one hand, by virtue of its properties for the deflection of fluid and, on the other hand, by virtue of its swelling-up during the absorption of fluid.

The increase in the quantity of fluid absorbed in a tampon according to the invention (absorption) and also 25 the dwell time of fluid in a tampon according to the invention (time) may also be gathered from the following table:

| Attribute | Standard Tampon | Tampon of 2.6 g with a second region of 0.4 g composed of: | | |
|---|---|---|---|---|
| | | Fibre mixture of 70% hydrophobic Danufil® and 30% hydrophilic Galaxy™ | Fibre mixture of 90% hydrophobic Danufil® and 10% hydrophilic Galaxy™ | 100% hydrophobic Danufil® |
| Absorption (ml) | 15.9 ± 0.3 | 17.2 ±1.7 | 16.3 ±1.1 | 15.0 ±1.0 |
| Time (sec) | 9.5 ± 1.2 | 11.4 ±1.2 | 11.4 ±1.5 | 10.4±0.9 |

It is accordingly particularly advantageous if the weight of the material of the second region 6 is between about 1/5 and about 1/6 of the total weight of the tampon 1 and comprises a fibre mixture which comprises about 70% by weight of hydrophobic fibres and about 30% by weight of hydrophilic fibres.

## Claims

1. Tampon having an introduction end and a recovery end disposed at opposite ends of a longitudinal axis, the tampon comprising:
a) a first region which extends from the introduction end toward the recovery end and comprises a first material, and
b) a second region which is located proximate the recovery end of the tampon and comprises a second material, the first material having a higher absorbency and a higher hydrophilicity than the second material; *wherein the second material comprises hydrophilic fibres and at least approximately 70 wt-% hydrophobic fibres,* wherein the diameter of the second region is at least as large as the mean diameter of the first region as measured essentially perpendicularly to the longitudinal axis, and during the absorption of fluid, the second region expands at least essentially perpendicularly to the longitudinal axis of the tampon.

2. Tampon according to claim 1, wherein *during the absorption of fluids* the expansion of the second region insures that the diameter of the second region perpendicularly to the longitudinal axis of the tampon is larger than that of the first region.

3. Tampon according to claim 1, wherein the second material further comprises fibre nonwovens.

4. Tampon according to anyone of claims 1 to 3, wherein the hydrophobic fibres comprise fibres having a hydrophobic finish.

5. Tampon according to anyone of claims 1 to 4, wherein the hydrophobic fibres comprise fibres selected from the group consisting of cellulose, polyolefin, polyester, and combinations thereof, the fibres having a hydrophobic finish.

6. Tampon according to one of the preceding claims, wherein the diameter of the second region is larger as the mean diameter of the first region, prior to use of the tampon.

7. Tampon according to one of the preceding claims, wherein the weight of the first material is at least approximately 4/5 of the total weight of the tampon.

8. Tampon according to one of the preceding claims, wherein the weight of the first material is at most at least approximately 5/6 of the total weight of the tampon.

9. Tampon according to one of the preceding claims, wherein the second region is at least partially fluid-permeable.

10. Tampon according to one of the preceding claims, wherein the first material comprises fibrous material selected from the group consisting of hydrophilic fibre material, mixtures of hydrophilic and hydrophobic fibres, fibre nonwovens and combinations thereof.

11. Tampon according to one of the preceding claims, wherein the first material comprises cellulose fibres.

12. Tampon according to one of the preceding claims, wherein during absorption of fluid, the first material doubles in volume.

13. Tampon according to one of the preceding claims which further comprises a withdrawal string extending from the recovery end.

## Patentansprüche

1. Tampon, der ein Einführungsende und ein Rückholende aufweist, die an gegenüberliegenden Enden einer longitudinalen Achse angeordnet sind, wobei der Tampon umfaßt:
a) einen ersten Bereich, der sich vom Einführungsende zum Rückholende hin erstreckt und ein erstes Material umfaßt, und
b) einen zweiten Bereich, der sich in der Nähe des Rückholendes des Tampons befindet und ein zweites Material umfaßt, wobei das erste Material ein höheres Absorptionsvermögen und eine höhere Hydrophilie als das zweite Material aufweist, *wobei das zweite Material hydrophile Fasern und einen Anteil an hydrophoben Fasern von zumindest zirka 70 Gew.-% umfaßt,* wobei der Durchmesser des zweiten Bereichs zumindest so groß wie der mittlere Durchmesser des ersten Bereichs ist, gemessen im wesentlichen senkrecht zur longitudinalen Achse, und wobei der zweite Bereich sich während der Absorption von Flüssigkeit zumindest im wesentlichen senkrecht zur longitudinalen Achse des Tampons erweitert.

2. Tampon nach Anspruch 1, **dadurch gekennzeichnet, daß** *während der Absorption von Flüssigkeiten* die Erweiterung des zweiten Bereichs gewährleistet, daß der Durchmesser des zweiten Bereichs senkrecht zur longitudinalen Achse des Tampons größer als der des ersten Bereichs ist.

3. Tampon nach Anspruch 1, **dadurch gekennzeichnet, daß** das zweite Material ferner Faservlies umfaßt.

4. Tampon nach einem der Ansprüche 1 bis 3, **dadurch gekennzeichnet, daß** die hydrophoben Fasern Fasern mit einem hydrophoben Finish umfassen.

5. Tampon nach einem der Ansprüche 1 bis 4, **dadurch gekennzeichnet, daß** die hydrophoben Fasern Fasern umfassen, ausgewählt aus der Gruppe, bestehend aus Zellstoff, Polyolefin, Polyester und Kombinationen derselben, wobei die Fasern ein hydrophobes Finish aufweisen.

6. Tampon nach einem der vorangehenden Ansprüche, **dadurch gekennzeichnet, daß** der Durchmesser des zweiten Bereiches größer ist als der mittlere Durchmesser des ersten Bereiches, vor dem Gebrauch des Tampons.

7. Tampon nach einem der vorangehenden Ansprüche, **dadurch gekennzeichnet, daß** das Gewicht des ersten Materials zumindest zirka 4/5 des Gesamtgewichts des Tampons ausmacht.

8. Tampon nach einem der vorangehenden Ansprüche, **dadurch gekennzeichnet, daß** das Gewicht des ersten Materials höchstens zumindest zirka 5/6 des Gesamtgewichts des Tampons ausmacht.

9. Tampon nach einem der vorangehenden Ansprüche, **dadurch gekennzeichnet, daß** der zweite Bereich zumindest teilweise flüssigkeitsdurchlässig ist.

10. Tampon nach einem der vorangehenden Ansprüche, **dadurch gekennzeichnet, daß** das erste Material Fasermaterial umfaßt, ausgewählt aus der Gruppe, bestehend aus hydrophilem Fasermaterial, Mischungen von hydrophilen und hydrophoben Fasern, Faservliesen und Kombinationen derselben.

11. Tampon nach einem der vorangehenden Ansprüche, **dadurch gekennzeichnet, daß** das erste Material Zellulosefasern umfaßt.

12. Tampon nach einem der vorangehenden Ansprüche, **dadurch gekennzeichnet, daß** während der Absorption von Flüssigkeit sich das Volumen des ersten Materials verdoppelt.

13. Tampon nach einem der vorangehenden Ansprüche, welcher weiterhin ein Rückholbändchen umfaßt, das sich von dem Rückholende erstreckt.

## Revendications

1. Tampon ayant une extrémité d'introduction et une extrémité de récupération disposées à des extrémités opposées d'un axe longitudinal, le tampon comprenant :
a) une première région qui s'étend de l'extrémité d'introduction à l'extrémité de récupération et comprend un premier matériau, et
b) une seconde région qui est située à proximité de l'extrémité de récupération du tampon et comprend un second matériau, le premier matériau ayant une capacité d'absorption supérieure et une capacité hydrophile supérieure à celles du second matériau, où *le second matériau comprend des fibres hydrophiles et au moins approximativement 70 % en poids* de *fibres hydrophobes,* où le diamètre de la seconde région est au moins aussi grand que le diamètre moyen de la première région tel que mesuré de manière essentiellement perpendiculaire par rapport à l'axe longitudinal, et au cours de l'absorption de fluide, la seconde région se dilate au moins de manière essentiellement perpendiculaire par rapport à l'axe longitudinal du tampon.

2. Tampon selon la revendication 1, dans lequel au *cours de l'absorption de fluides,* la dilatation de la seconde région garantit que le diamètre de la seconde région de manière perpendiculaire par rapport à l'axe longitudinal du tampon soit plus grand que celui de la première région.

3. Tampon selon la revendication 1, dans lequel le second matériau comprend en outre des fibres non tissées.

4. Tampon selon l'une quelconque des revendications 1 à 3, dans lequel les fibres hydrophobes comprennent des fibres ayant un apprêt hydrophobe.

5. Tampon selon l'une quelconque des revendications 1 à 4, dans lequel les fibres hydrophobes comprennent des fibres choisies dans le groupe constitué de cellulose, polyoléfine, polyester, et leurs combinaisons, les fibres ayant un apprêt hydrophobe.

6. Tampon selon l'une quelconque des revendications précédentes, dans lequel le diamètre de la seconde région est plus grand que le diamètre moyen de la première région, avant utilisation du tampon.

7. Tampon selon l'une quelconque des revendications précédentes, dans lequel le poids du premier matériau représente au moins approximativement 4/5ème du poids total du tampon.

8. Tampon selon l'une quelconque des revendications précédentes, dans lequel le poids du premier matériau est au maximum d'au moins approximativement 5/6ème du poids total du tampon.

9. Tampon selon l'une quelconque des revendications précédentes, dans lequel la seconde région est au moins partiellement perméable aux fluides.

10. Tampon selon l'une quelconque des revendications précédentes, dans lequel le premier matériau comprend un matériau fibreux choisi dans le groupe constitué d'un matériau à fibre hydrophobe, de mélanges de fibres hydrophiles et hydrophobes, de fibres non tissées et de leurs combinaisons.

11. Tampon selon l'une quelconque des revendications précédentes, dans lequel le premier matériau comprend des fibres de cellulose.

12. Tampon selon l'une quelconque des revendications précédentes, dans lequel au cours de l'absorption d'un fluide, le premier matériau double de volume.

13. Tampon selon l'une quelconque des revendications précédentes, lequel tampon comprend en outre un cordon de retrait qui s'étend depuis l'extrémité de récupération.
